# EUROPEAN PATENT APPLICATION

(11) **EP 2 014 316 A1**
(43) Date of publication of application: **14.01.2009**
(21) Application number: 07742280.6
(22) Date of filing: 24.04.2007
(51) Int. Cl.: A61L 27/00

(54) **METHOD OF PREPARING ORGAN FOR TRANSPLANTATION**

(30) Priority: 24.04.2006 JP 2006118799
(71) Applicant: Stemcell Institute Inc., Tokyo 105-0004 (JP)
(72) Inventor: FUKUI, Akira, Minato-ku, Tokyo 105-8461 (JP); YOKOO, Takashi, Minato-ku, Tokyo 105-8461 (JP); OKABE, Masataka, Minato-ku, Tokyo 105-8461 (JP); HOSOYA, Tatsuo, Minato-ku, Tokyo 105-8461 (JP)
(74) Representative: Krauss, Jan
(86) International application number: PCT/JP2007/058845
(87) International publication number: WO 2007/125916

(57) **Abstract**

The present invention provides a method for preparing an organ, particularly a kidney, for transplantation into mammals. In detail, the present invention provides a method for preparing autotransplantation of autologous organs, particularly a kidney, wherein the isolated autologous mesenchymal stem cells are transplanted into an embryo inside a pregnant mammalian host or into an embryo dissected from a pregnant mammalian host at a desired site to induce differentiation, which is then transplanted into the individual.

## Description

### Technical field

The present invention provides a method for preparing an organ, particularly a kidney, for transplantation into mammals. Additionally, the present invention files a priority from Japanese Patent Application Number 2006-118799, is incorporated herein by reference.

### Background Art

Organ regeneration has recently attracted considerable attention as a new therapeutic strategy. The potential for regenerative medicine has been gradually realized with the discovery of various tissue stem cells, and with reports on therapeutic benefits through the regeneration of neurons (non-patent document 1), β cells (non-patent document 2), myocytes (non-patent document 3), blood vessels (non-patent document 4) and the like using tissue stem cells. However, successful examples using such strategies to date have been limited to the cells and simple tissues. Anatomically complicated organs such as the kidney and lung, which are comprised of several different cell types and have a sophisticated 3-dimentinal organization and cell signaling system, have especially proven more refractory to stem cell-based regenerative techniques.

With advances in medical transplantation, these complex organs have been expected to be transplanted to bring about the complete recovery of a seriously damaged organ. However, there is a worldwide chronic shortage of donors. Furthermore, even successful transplantation needs a long-term administration of immunosuppressive drugs to avoid the rejection reaction, compelling recipient to continue suffering from the accompanying side-effects (non-patent document 5).

Therefore, one of the ultimate therapeutic aims is to establish self-organs from autologous tissue stem cells and to transplant the in vitro-derived organ as a syngraft back into the donor individual. Human mesenchymal stem cells (hMSCs) found in adult bone marrow have been recently made known to maintain plasticity and to differentiate into several different cell types, depending on their microenvironment (non-patent document 6). In contrast to embryonic stem cells (ES cells), hMSCs can be isolated from autologous bone marrow and be applied for therapeutic use without any serious ethical issues or immunologic consequences (non-patent document 7).
[Non-patent document 1] J. Neurosci. Res. 69,925-933(2002)
[Non-patent document 2] Nat. Med. 6, 278-282(2000)
[Non-patent document 3] Nature 410,701-705(2001)
[Non-patent document 4] Nat. Med. 5, 434-438(1999)
[Non-patent document 5] Transplantation 77, S41-S43(2004)
[Non-patent document 6] Science 276, 71-74(1997)
[Non-patent document 7] Birth Defects Res. 69, 250-256(2003)
[Non-patent document 8] Organogenesis of the Kidney (Cambridge Univ. Press, Cambridge, U.K.) (1987)
[Non-patent document 9] Exp. Nephrol. 10, 102-113(2002)
[Non-patent document 10] Am. J. Kidney Dis. 31, 383-397(1998)
[Non-patent document 11] J. Neurosci. Res. 60, 511-519(2000)
[Non-patent document 12] Blood 98, 57-64 (2001)
[Non-patent document 13] J. Am. Soc. Nephrol. 11, 2330-2337(2001)
[Non-patent document 14] Methods 24, 35-42(2001)
[Non-patent document 15] J. Clin. Invest. 105, 868-873(2000)
[Non-patent document 16] J. Neurol. Sci. 65, 169-177(1984)
[Non-patent document 17] Kidney Int. 64, 102-109(2003)
[Non-patent document 18] Cytometry 12, 291-301(1991)
[Non-patent document 19] Dev. Growth Differ. 37, 123-132(1995)
[Non-patent document 20] Am. J. Physiol. 279, F65-F76(2000)
[Non-patent document 21] Eur. J. Physiol. 445, 321-330(2002)
[Non-patent document 22] Proc. Natl. Acad.Sci. USA 97, 7515-7520(2000)
[Non-patent document 23] Nature 418, 41-49(2002)
[Non-patent document 23] Am. J. Physiol. 280, R1865-1869(2001)
[Non-patent document 24] Methods 24, 35-42(2001)

### Disclosure of the Invention

### [Problem to be Solved by the Invention]

An object of the present invention is to provide a means for achieving the creation of a complex organ such as a kidney through the use of mammal-derived mesenchymal stem cells.

### [Means for Solving the Problem]

One of the target organs of the present invention is a kidney. The kidney represents a complex organ, comprising several different cell types, and having sophisticated and three-dimensional structures, and its developmental processes inside an embryo have been well researched. Kidney development is initiated when the metanephric mesenchyme at the caudal portion of the nephrogenic cord (non-patent document 8) induces the nearby Wolffian duct to produce a ureteric bud (non-patent document 9). Development proceeds as a result of reciprocal epithelial-mesenchymal signaling between the ureteric bud and metanephric mesenchyme (non-patent document 10). To test whether mammal-derived mesenchymal stem cells could participate in kidney development, human mesenchymal stem cells (hMSCs) were initially co-cultured either with rodent Wolffian duct extracted at the embryonic stage immediately before formation of the kidney primordia, or with established metanephric rudiment. However, this procedure was not sufficient to achieve kidney organogenesis or even integration of hMSCs into the developing rodent metanephros. This study suggests that hMSCs have to be placed in a specific embryonic niche to allow for exposure to the repertoire of signals required for the generation of the organ. The present inventors have discovered that the organogenesis can best be achieved by implanting hMSCs into the nephrogenic site of a developing embryo, and have completed one of the present inventions. That is to say, the mesenchymal stem cells can be differentiated into mesangium cells, renal tubular epithelial cells, and glomerular epithelial cells by transplanting themselves into a metanephros-forming mesenchyme. Further, the mesenchymal stem cells can be differentiated into a ureteric bud-derived collecting tube and ureter by transplanting themselves into intermediate mesoderm.

It is difficult to transplant cells prenatally at the exact site of organogenesis by a transuterine approach. In addition, once embryos are removed for cell transplantation, they cannot be returned to the uterus for further development. The present inventors have isolated embryos from uteri for cell transplantation, after which the embryos were developed in vitro through whole-embryo culture until the embryos ended the initial stage of organogenesis, and further matured the embryos in organ culture and the abdominal cavity of a recipient. In the rest of the present invention, the present inventors have found that by using this culture combination, hMSCs differentiate into morphologically identical cells to endogenous renal cells and are able to contribute to complex kidney structures. Furthermore, the present inventors have shown that this novel kidney has a filtering function and can receive the bloodstream from the recipient and generate urine, and have completed the present invention.

More specifically, the present invention includes:
1. A method for preparing an organ for transplantation into a mammal by transplanting isolated mammal-derived mesenchymal stem cells into the embryo inside a pregnant mammal host or into the embryo dissected from a pregnant mammal host to induce differentiation of the mesenchymal stem cells, wherein the mesenchymal stem cells are transplanted into intermediate mesoderm of the embryo, at a transplantation time when the host is still at an immunologically tolerant stage.
2. The method according to item 1, wherein the above organ is a kidney.
3. The method according to item 2, wherein the above mammal-derived mesenchymal stem cells are differentiated into mesangium cells, tubular epithelial cells, and glomerular epithelial cells by separately transplanting the mesenchymal stem cells into a metanephros-forming mesenchyme.
4. The method according to any one of items 1 to 3, wherein the mammal-derived mesenchymal stem cells are differentiated into the mammal-derived ureteric bud-derived collecting tube and ureter by transplanting the mesenchymal stem cells into the intermediate mesoderm.
5. The method according to any one of items 1 to 4, wherein the above host is a mammal having a size of the kidney similar to that of the human kidney.
6. The method according to item 5, wherein the above host is a pig.
7. The method according to any one of items 1 to 6, wherein the mammal-derived mesenchymal stem cells are transplanted into an embryo by transplanting the cells to the host through a transuterine approach.
8. The method according to any one of items 1 to 7, wherein the mammal-derived mesenchymal stem cells are transplanted into an embryo by dissecting the embryo from the uterus and transplanting the cells into that embryo, and then further maturing the embryo in vitro using whole embryo culture.
9. The method according to any one of items 1 to 8, wherein the mammal-derived mesenchymal stem cells are human mesenchymal cells.

### [Effects of the invention]

The present invention provides a novel means for autotransplantation of autologous organs, particularly a kidney. In other words, the isolated mesenchymal stem cells of an individual can be transplanted into an embryo inside a pregnant mammalian host or into an embryo isolated from a pregnant mammalian host at a desired site to induce differentiation into the kidney, which is then transplanted to the individual.

### Brief description of the drawings

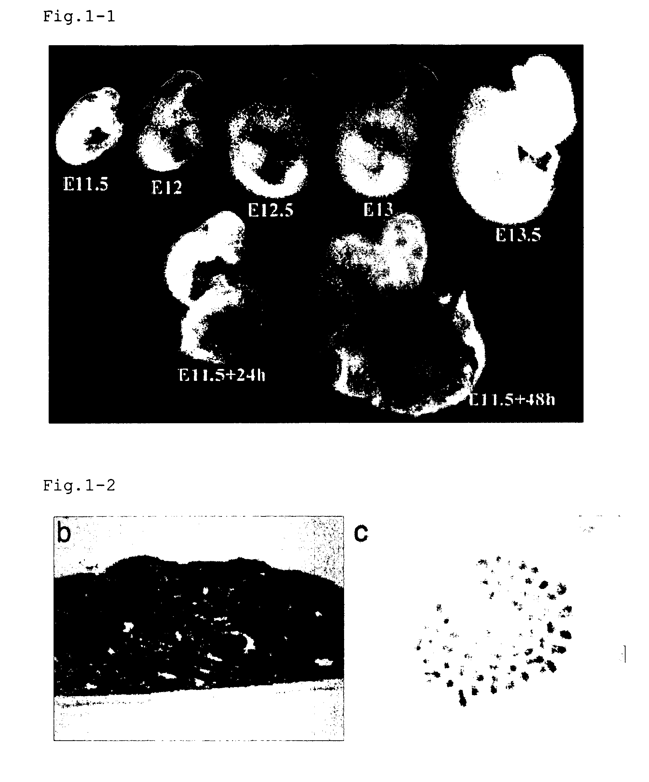

Fig. 1-1 is a figure showing the *ex utero* differentiation of the kidney primordia using a relay culture.
Fig. 1-2 is a figure showing the *ex utero* differentiation of the kidney primordia using the relay culture. In order to confirm the extent of tubule formation and ureteric bud branching, hematoxylin/eosin staining (b) and whole-mount *in situ* hybridization for c-ret (c) are performed.
Fig. 2 is a figure showing the proportion of donor-derived cells in the culture-derived metanephros and the assessment of its DNA-ploidy. "M" is the large informative peak.
Fig. 3-1 is a figure showing the differentiation of transplanted hMSCs into organized, resident renal cells. After relay culturing, the resulting metanephros was subjected to an X-gal assay to trace the transplanted hMSCs.
Fig. 3-2 is a figure showing the differentiation of transplanted hMSCs into organized, resident renal cells. (b) Serial sections were examined by optical microscopy. (c) Tissue sections were subjected to two-color immunofluorescent staining for beta-gal (left) and WT-1 (right).
Fig. 3-3 is a figure showing the differentiation of transplanted hMSCs into organized, resident renal cells. After relay culturing, the resulting metanephros were digested, and single cells were subjected to the FACS-galactosidase assay.
Fig. 4 is a figure showing the injection and culture of hMSCs in isolated metanephros. (a) After 6 days of organ culture, the resulting metanephros were subjected to an X-gal assay. (b) RNAs were extracted and subjected to RT-PCR.
Fig. 5-1 is a figure showing a therapeutic kidney regeneration in an alpha-gal A-deletion Fabry mouse. The alpha-gal A enzymatic bioactivity of resulting metanephros was fluorometrically assessed as described.
Fig. 5-2 is a figure showing a therapeutic kidney regeneration in an alpha-gal A-deletion Fabry mouse. To confirm the potency of the Gb3 clearance in resulting metanephros, organ culture was performed in the presence of Gb3, and accumulation in the metanephros was assessed by immunostaining for Gb3.
Fig. 6 is a figure showing the emergence of the metanephros transplanted in the greater omentum.
Fig. 7 is a figure showing the histological analysis of the metanephros (2 weeks) transplanted inside the greater omentum.
Fig. 8 is a figure showing transplantation (2 weeks) of different stages of kidney primordial into the greater omentum.
Fig. 9 is a figure showing the new kidney generated with improved relay culture (2 weeks).
Fig. 10 is a figure showing that the vascular inside the new kidney is constructed from the recipient.
Fig. 11 is a figure showing an electron microscope photograph of the new kidney transplanted into the greater omentum.
Fig. 12 is a figure showing the histological findings of the new kidney created by the improved relay culture (2 weeks) in LacZ rat from LacZ-positive human mesenchymal stem cells.
Fig. 13 is a figure showing the new kidney produced by the improved relay culture (4 weeks).
Fig. 14 is a figure showing the liquid-like urine from the new kidney.
Fig. 15 is a figure showing the trace of the movement of the material labeled the intermediate mesoderm, by observing with the lapse of time using a fluorescent stereoscopic microscope.

### Description of the Preferred Embodiment

The present invention is the method for preparing a kidney for transplantation into mammals, particularly human, by transplanting isolated mammal-derived mesenchymal stem cells, particularly human mesenchymal stem cells (hMSCs), into the embryo inside a pregnant mammal host or into the embryo dissected from a pregnant mammal host to induce differentiation.

Through the method for preparing an organ for transplantation of the present invention, a organ of mammals including, for example, humans, pet animals such as monkeys, cows, sheep, pigs, goats, horses (particularly, racing horses), dogs, cats, rabbits, hamsters, guinea pigs, rats, or mice can be prepared. The suitable example of the host is a pig, and other suitable animals include genetically modified pigs such as transgenic, knockout, or knock-in pigs. Other examples include ungulates such as cows, sheep, pigs, goats, and horses. Further suitable examples include genetically modified animals such as mice or the above-mentioned ungulates, particularly transgenic animals.

It is preferable that mesenchymal stem cells (MSCs) are derived from the recipient of transplanted target. For example, when the recipient is human, mesenchymal stem cells are isolated from bone marrow, circulating blood, or cord blood in human. It is preferable that the mesenchymal stem cells (MSCs) are isolated from bone marrow, circulating blood, or cord blood of the recipient himself or herself. The preparative isolation is performed by a general surgical procedure. The isolated cells are cultured under a selected optimal condition, but the passage number is preferably within 2-5. The culture medium kit for human mesenchymal stem cells manufactured by Cambrex BioScience is more preferably used to keep culturing the hMSCs without being transformed. If desired, the mesenchymal stem cells are transfected with a desired gene by the manipulation using, for example, adenovirus and/or retrovirus. For example, if a kidney is desired to provide, the cell is transducted with a gene in order to express the glial cell line-derived neurotrophic factor (GDNF) for assisting the formation of the kidney. This is because the transfection facilitates the mesenchymal tissue to express GDNF immediately before kidney formation so that the ureteric bud expressing the c-ret, a receptor for the factor is taken into the process to complete the first important step for kidney generation. Through this transfection, it is confirmed that the formation rate of an injected stem cell-derived kidney is increased from 5.0±4.2% to 29.8±9.2%.

The prepared mesenchymal stem cells are then transplanted into an embryo inside a pregnant mammalian host animal. Namely, the MSCs are directly transplanted into the embryo inside the body to form the kidney inside the uterus. The transplantation is performed by general surgical methods, for example, by using a micropipette while examining under echo. The cellular quantity of 0.5 X 10³ to 1.0 X 10³ is sufficient for the transplantation. Namely, the mesenchymal stem cells are directly transplanted by a transuterine approach into the embryo inside the live body of a large pregnant mammal such as a pig, and left to grow inside the live body into a kidney for transplantation. Further, the processes of "whole-embryo culture" or "organ culture" can be then added as described below, but their addition is not especially necessary because of the sufficient growth of the kidney for transplantation.

Additionally, the prepared hMSCs are preferably transplanted into embryos isolated from pregnant mammalian host animals (uteri), and afterward the embryos were developed in vitro through whole-embryo culture until the embryos ended the initial stage of organogenesis (kidney for transplantation), further cultured through organ culture, and the kidneys for transplantation are completed. Furthermore, the kidney for transplantation is transplanted into the greater omentum of mammals including a human.

The time for the transplantation of the prepared hMSCs into the embryos is selective. The experiments using rats were preferably E9 to 12, more preferably E10 to 12, still more preferably E10 to 11.5. Even in a large mammal such as a pig, the similar embryonic stage is suitable. However, by selecting appropriate conditions, an earlier or later stage can also be selected. In any case, it is important that the cells should be transplanted into the embryo at least at a time when the host is still at an immunologically tolerant stage.

Further, the host immune system is not yet fully developed at this stage of the whole-embryo culture. Therefore, the host is tolerant to foreign cells. The present invention has established a method for generating self-organs from autologous mesenchymal stem cells using the endogenous development system of an immunocompromised foreign host.

The feature of the present invention is to select the transplantation site where mesenchymal stem cells are transplanted into embryos. In other words, the transplantation site of the mesenchymal stem cells into the embryo is the corresponding site for generation of the kidney in the host. Therefore, the cells must be transplanted at a site when the site can be confirmed to be the corresponding site of the kidney, but it is preferable that the bud cells in the kidney are in a sprouting state prior to starting development. For example, the mesenchymal stem cells can be differentiated into mesangium cells, renal tubular epithelial cells, and glomerular epithelial cells by transplanting into the metanephros-forming mesenchyme. The mesenchymal stem cells can be differentiated into a ureteric bud-derived collecting tube and ureter by transplanting into the intermediate mesoderm.

"Whole-embryo culture" of the present invention is performed when hMSCs are transplanted into the embryos dissected from pregnant mammalian host animals (uteri). The outline of whole-embryo culture is that uteri are dissected from mothers, and hMSCs are transplanted into embryos freed from the uterine wall, decidua, and the outer-membrane layer, including Reichert's membrane, and then embryos are cultured in a culture bottle or the like. If the aim of culturing the kidney for transplantation of the present invention can be achieved with "whole-embryo culture," some improvement may be introduced and/or some process may be deleted in the aforementioned culture method. The following is provided to illustrate in more detail but is not limited to this. Whole embryos were cultured *in vitro* according to a previously described method (non-patent document 24), with several modifications. Using a surgical microscope and the like, uteri were dissected from anaesthetized mothers. The rat embryos which are preferably E9-12, specially E10-12, more preferably E10-11.5, and still more preferably E11.5 are freed from the uterine wall, decidua, and the outer-membrane layer, including Reichert's membrane. The yolk sac and amnion are opened to allow the injection of the hMSCs, but the chorioallantoic placenta is left intact. The embryos confirmed as success in injection of the hMSCs were cultivated in the culture bottles containing 3ml of culture media (glucose, penicillin G, streptomycin, and streptomycin and amphotericin B) comprising of centrifuged rat serum. The culture bottles are allowed to rotate in an incubator (model no. RKI10-0310, Ikemoto, Tokyo). Culture time is preferable 12-60 hours, more preferable 24-48 hours, and still more preferable 48 hours. Furthermore, after a certain culture time, the embryo is preferably assessed in terms of morphology and function, and the organ primordia for transplantation of kidney are confirmed. After this confirmation, the organ primordia are separated from the embryo to preferably carry out organ culture according to the following method.

The outline of "organ culture" of the present invention is that the above organ primordia are placed on a filter and added DMEM on the dish under them. The dish is incubated in incubator under condition of 5% CO₂. The culture time is preferably 12 to 168 hours, more preferably 18 to 72 hours, still more preferably 24 to 48 hours, and most preferably 24 hours. Accordingly, it is the most effective that the organ primordia are transplanted at the culture time of about 24 hours into the greater omentum. Additionally, the cultivation temperature is preferably 20 to 45°C, more preferably 25 to 40°C and most preferably 37°C. If the aim of transplanting the kidney for transplantation of the present invention can be achieved with "organ culture, some improvement may be introduced and/or some process may be deleted in the aforementioned culture. J. Clin. Invest.105, 868-873(2000) (non Patent document 15) is provided to illustrate in detail but is not to be construed as limiting the scope thereof.

The outline of "relay culture" of the present invention is that the above whole embryo culture is performed for 2 to 60 hours, and next the above-mentioned organ culture is performed for 12 to 168 hours. Furthermore, the outline of "improved relay culture" of the present invention is that the above whole embryo culture is performed for 2 to 60 hours, and next the above-mentioned organ culture is performed for 12 to 36 hours and further transplantation of greater omentum is performed.

Since the size of obtained organ is the same as that of the organ of the host animal, for example, the host is preferably a mammal having a similar size to the organ of human in order to create the kidney enough to fulfill adequate function in human. However, the host does not necessarily have exactly the same size of the organ. For example, even an obtained kidney, which has as little as 1/10^{th} of the perfect function, works adequately to carry out dialysis and is sufficient to sustain life. For this reason, pigs are the optimal hosts, and even miniature pigs have an adequate organ size to exhibit the function in human.

The kidney thus grown is, after the confirmation of its function, is then dissected from the host, and donated to the recipient, and is transplanted into the greater omentum of the recipient as one of preferred sites. The transplanted kidney continues developing in the body, and completes the formation of a cloned kidney which fulfills renal function. The method for "transplanting the kidney into a greater omentum of mammals including a human" of the present invention is performed by a general surgical procedure. For example, a tissue for transplantation is anchored with a sharp tweezers, small incision is made over the surface of an adipose tissue of a greater omentum with the tip of the tweezers, and the tissue is implanted in the incision. Moreover, the kidney for transplantation can be transplanted into the greater omentum with an endoscope.

In order that the formed kidney may not be contaminated with antigenic substances from the host as foreign substances, the transformation of transplanted cells as follows is effective. Namely, the formed kidney contains a coexistence of the mesenchymal stem cells-derived cells and the host animal-derived cells. When the kidney is transplanted into the recipient, the host-derived cells in coexistence are likely to induce an immunological rejection reaction and thus have to be completely removed after the formation of the kidney. In order to solve this problem, the host animal designed to induce controllable programmed cell death is produced and then allowed to form the kidney. The mesenchymal stem cells are transplanted into the corresponding site of the embryo of the host animal to form a kidney, which is then allowed to induce cell death specific to the host cell, thereby to clear completely of the host-derived cells at a step prior to transplantation into a recipient.

### [Examples]

As a representative example of the present invention, a system for a kidney using rat will be described. However, the present invention is not limited to this system.

### Example 1

### (Materials and methods)

### 1) Experimental animals

The animals used were wild-type Sprague-Dawley rats purchased from Sankyo Lab Service (Tokyo). At the Laboratory Animal Center of the Jikei University School of Medicine, a breeding colony of Fabry mice was established from breeding pairs donated by Mr. R.O. Brady (National Institute of Health, Bethesda). The midpoint where a vaginal plug was seen was designated as day 0.5. Animals were housed in a ventilated (positive pressure airflow) rack and were bred and raised under pathogen-free conditions. All experimental procedures were approved by the Committee for Animal Experiments of the Jikei University School of Medicine.

### 2) Culture and manipulation of hMSCs

hMSCs obtained from the bone marrows of healthy volunteers were used. Bone marrow-derived hMSCs confirmed to be CD105-, CD166-, CD29-, CD44-positive, and CD 14-, CD34-, CD45- negative were purchased from Cambrex BioScience Co. (Walkersville, MD). Following the protocol provided by the manufacturer, these hMSCs were cultured. In order to avoid phenotypical changes, the hMSCs were used within five cell passages. A replication-defective recombinant adenovirus carrying human glial cell line-derived neurotrophic factor GDNFcDNA (AxCAhGDNF) was generated and purified as described (non-Patent document 11). Packaging cells (Ψ-crip) that produce a recombinant retrovirus having the bacterial LacZ gene (MFG-LacZ) were donated by H. Hamada (Sapporo Medical University, Sapporo, Japan). Adenoviral and retroviral infections were performed as described (non-patent documents 12, 13). The cells were labeled with 1,1'-dioctadecyl-3,3,3',3'-tetramethylindocarbocyanine (DiI) (Molecular Probes) at 0.25% (wt/vol) in 100% dimethylformamide and were injected into the sprouting site of ureteric bud by using micropipettes.

### 3) Whole-embryo culture and organ culture

Whole embryos were cultured *in vitro* according to a previously described method (non-patent document 14), with several modifications. Using a stereoscopic microscope, uteri were dissected from anaesthetized mothers. (E: stage embryonic day) E11.5 rat embryos and E9.5 mouse embryos were freed from the uterine wall, decidua, and the outer-membrane layer, including Reichert's membrane. The yolk sac and amnion were opened to allow injection, but the chorioallantoic placenta was left intact. Successfully injected embryos were immediately cultivated in 15-ml culture bottles containing 3 ml of culture media consisting of 100% centrifuged rat serum supplemented with glucose (10mg/ml), penicillin G (100 units/ml), streptomycin (100 micrograms/ml), and amphotericin B (0.25 micrograms/ml). The culture bottles were allowed to rotate in an incubator (model no. RKI10-0310, Ikemoto, Tokyo). *Ex vivo* development of the rat embryos was assessed after 24 to 48 hour culture periods and compared with E 12.5 and E 13.5 rat embryos. Forty-eight hours later, embryos were assessed in terms of heartbeat, whole-body blood circulation, and general morphology. Kidney primordias were dissected and cultured as described previously (non-patent document 15). To enhance the accumulation of globotriaosylceramide (Gb3) in the kidney primordia, the cultivated metanephros were cultured in the presence of ceramide trihexoside (1 nmol, Sigma) (non-patent document 16). Alpha-galactosidase A (alpha-gal A) enzymatic activity in metanephros was fluorometrically assessed as described (non-patent document 17).

### 4) Histology

Two-color staining of metanephros was performed essentially as described (non-patent document 17) by using mouse anti-beta-gal (Promega) and rabbit anti-human WT-1 (Santa Cruz Biotechnology) as primary antibodies. A monoclonal mouse anti-Gb3 antibody (Seikagaku, Tokyo) was also used.
Whole-mount *in situ* hybridization with digoxigenin UTP-labeled *c-ret* riboprobes was performed as described (non-patent document 15). *In situ* hybridization was also performed on histological sections by using biotin-labeled human genomic Alul/II probes (Invitrogen) according to the manufacturer's protocol. An X-gal assay was used to assess expression of the LacZ gene as described (non-patent document 13).

### 5) Identification of hMSC-derived LacZ-positive cells

Metanephros generated by relay culture were digested in collagenase type I (1 mg/ml) for 30 min and were labeled with fluorescein digalactoside (Molecular Probes) by making use of transient permeabilization through hypotonic shock (non-patent document 18) (FACS-Gal assay). LacZ-positive cells were sorted using a cell sorter (Becton Dickinson). Total RNA was extracted and subjected to RT-PCR to analyze expression of aquaporin-1 (AQP-1), parathyroid hormone (PTH) receptor 1, 1 alpha hydroxylase, Na⁺-HCO₃⁻ co-transporter 1 (NBC1), nephrin, podocine, and glomerular epithelial protein 1 (GLEPP-1). For the analysis of cell ploidy, cells were stained with propidium iodide, and DNA content was assessed by using a flow cytometer.

### 6) Creation of functional donor-derived clone kidney

In order to study the optimal conditions for the growth of the kidney primordia inside the greater omentum, the degree of growth after transplantation was evaluated by the growth stage of the rat metanephros tissue and presence or absence of heminephrectomy. In accordance with the optimal conditions, the kidney primordia created as described above were further transplanted into the greater omentum of the recipient. After 2 weeks, whether there were findings of highly differentiated tissues of the kidney was confirmed by immunologic staining and electron microscopy.

### 7) Confirmation of the integration of the blood vessels of the recipient and clone kidney

In order to confirm that there was blood flow of the recipient to the new kidney, the kidney was transplanted into the greater omentum of LacZ transgenic rat. It was confirmed that the blood vessels inside the new kidney were derived from the recipient. The human mesenchymal stem cells further injected also introduced the LacZ gene, and whether the blood vessels and the donor derived-nephrons were integrated, was confirmed.

### 8) Confirmation of the presence or absence of urine generating function

In order to study whether the new kidney, which was grown in the greater omentum and which had circulation of recipient's blood, can filter the recipient blood and generate urine, the kidney was developed for 4 weeks inside the greater omentum, and the urea nitrogen concentration and creatinine concentration in the liquid collected inside the ureter were measured and compared with the serum concentration to confirm the presence or absence of urine generating capability.

### 9) Statistical analysis

Data were expressed as the mean ± standard deviation. Statistical analysis was performed by using the two-sample *t* test to compare data in different 2 groups. P<0.05 was taken to be statistically significant.

### (Results)

A. *Ex-utero* development of kidney primordia by using the relay culture system The whole-embryo culture system was optimized to allow a defined concentration of oxygen to be supplied continuously to rotating culture bottles, and thus embryonic development ex utero was improved (non-patent document 14). Using this system, rat embryos (E11.5) were cultured at 37°C in the culture bottle consisting of the media composed of 100% freshly centrifuged rat serum supplemented with glucose (10 mg/ml), together with the yolk sac, amnion, and chorioallantoic placenta. After 24 and 48 hours in culture, *ex utero* development of the rat embryos was assessed by comparing with those that grown *in utero* for E11.5, E12.0, E12.5, E13.0, and E13.5. Forty-eight hours later, embryos were assessed in terms of heartbeat, whole-body blood circulation, and general morphology. Based on the resultant somite number and general morphology, the developmental age of rat embryos cultured through this method appeared consistent with E13 embryos that had developed *in utero* (Fig. 1-1). At this stage, ureteric buds were elongated and initial branching was completed, indicating that during culture, the metanephric mesenchyme had been stimulated to take the first step toward nephrogenesis. However, embryos could not develop further and died soon after 48 hours because of insufficient development of the placenta *in vitro* (non-patent document 19). To overcome this limitation, whole-embryo culture was followed by organ culture. After whole-embryo culture for 48 hours, metanephros were dissected from embryos and subjected to organ culture for 6 days. Using this combination (relay culture), kidney primordias continued to differentiate and grow *in vitro.* Repeated tubule formation and ureteric bud branching were confirmed by performing hematoxylin/eosin staining (Fig. 1-2(b)) and whole-mount *in situ* hybridization for c-ret (Fig. 1-2(c)). This shows that the metanephros can complete development *ex utero,* even if the embryo is dissected from the uterus before the stage at which the ureteric bud sprouts.

### B. Proportion of donor-derived cells in culture-derived metanephros and assessment of the possibility of cell fusion

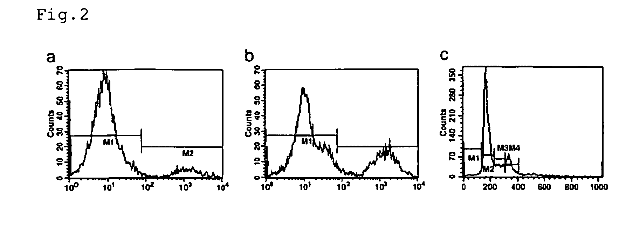

Using the system described in A, hMSCs were injected into rat embryos at the kidney-forming site. In order to distinguish the hMSCs from the host-derived cells, the hMSCs was forced to express the LacZ gene using retrovirus, and the hMSCs labeled with DiI fluorescent were injected into the budding site of the ureteric bud of the rat embryo using adenovirus transducted with GDNF (Fig. 2(b)) or without (Fig. 2(a)). Next, a total of 1 x 10³/embryo of hMSCs were then injected into the intermediate mesoderm between the somite and the lateral plate at the level of somite 29 for rat and somite 26 for mouse. The present inventors previously estimated these levels, by *in situ* hybridization for c-ret, to be the ureteric budding sites (non-patent document 15). Successful injection was confirmed by the fact that injected hMSCs-derived cells were detected along the Wolffian duct by in situ hybridization for human genomic AluI/II which identifies exclusively human cells.

After relay culture, the newly generated kidney primordia were digested with collagenase, and when single cells were subjected to FACS-Gal assay, 5.0±4.2% of LacZ-positive cells were detected in the kidney primordium tissue (Fig. 2(a)). No LacZ-positive cells were detected in the isolated metanephros when the injection site was altered by over 1 somite in length. In control embryos, injection of labeled mouse fibroblasts instead of hMSCs resulted in an almost negligible number of LacZ-positive cells detected in the metanephros. To enhance the number of injected donor-derived cells, the hMSCs before injection were further modified to temporally express GDNF by using the adenovirus AxCAh-GDNF (Non-patent document 11). This is because GDNF is normally expressed in metanephric mesenchyme at this stage, and through the interaction between GDNF and its receptor, c-ret, epithelial-mesenchymal signaling is essential for the kidney formation (Non-patent document 10). The FACS-gal assay revealed a significant increase in the number of donor-derived LacZ-positive cells detected in the kidney through this transient GDNF expression (29.8±9.2%, Fig. 2(b)). When LacZ-positive cells were sorted, and their DNA content was assessed by using propidium iodide intensity, 68.8±11.4% of LacZ-positive cells in the neogenerated kidney primordium was euploid (Fig. 2(c)). In addition, the number of LacZ-positive cells was significantly increased (2.84±0.49 x 10⁵/kidney primordium) compared with the starting number of injected cells (1 x 10³/embryo), which suggests that the remaining polyploid cells were mostly undergoing cell division. Furthermore, fluorescent in situ hybridization using the human and rat Y chromosome showed no cells having two or more Y chromosomes were identified. These data strongly suggest that it is extremely unlikely that there will be cell fusion of host cell and donor cell.

### C. Differentiation of transplanted hMSCs into kidney cells

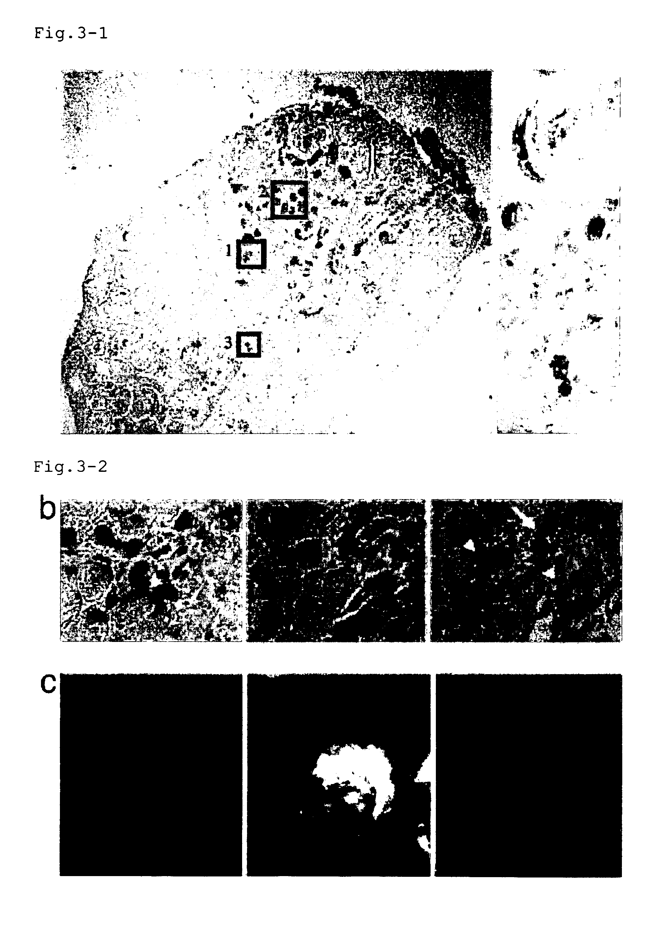

After relay culturing, the migration and morphologic changes of the hMSCs transplanted in the resulting kidney primordia were traced. In the organ culture, when the kidney primordia during growth were observed over time under a fluorescent microscope, DiI positive hMSCs migrated towards the medulla, and an image of these cells dispersing in the kidney primordia was confirmed. In order to study whether these cells contributed to renal structures, the kidney primordia were subjected to an X-gal assay. LacZ-positive cells were scattered throughout the metanephric rudiment and were morphologically identical to glomerular epithelial cells (panel 1), renal tubular epithelial cells (panel 2), and interstitial cells (panel 3) (Fig. 3-1). Furthermore, examination of serial sections of kidney primordia under a light microscope showed glomerular epithelial cells linked to tubular epithelial cells (arrow), and some of these cells formed a continuous tubular extension toward the medulla (arrow) (Fig. 3-2(b), gl: glomerulus). This image not only indicates that, after transplantation, the hMSC differentiates into individual kidney cells, but also indicates the formation of nephrons (the basic unit for filtration and reabsorption). For further confirmation of differentiation into glomerular epithelial cells, two-color immunofluorescent staining for beta-gal (left) and WT-1 (right) was conducted. WT-1 is known to be strongly expressed in glomerular epithelial cells at this stage (non-patent document 20). Since both were positive for the identical cells (center), this shows that some of LacZ-positive donor cells have completed differentiation to glomerular epithelial cells (Fig. 3-2(c)).

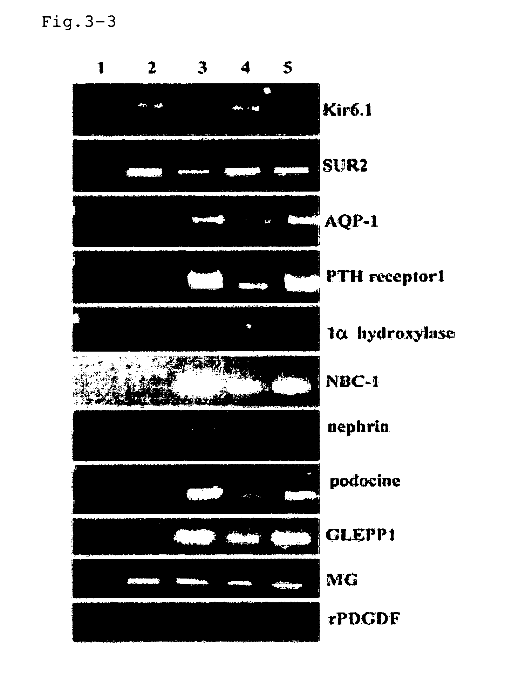

After relay culture, the resulting kidney primordia were digested, and single cells were subjected to the FACS-gal assay. LacZ-positive cells were sorted and subjected to RT-PCR for expression analysis of Kir6.1, SUR2, AQP-1, PTH receptor 1, 1 alpha hydroxylase, NBC-1, nephrin, podocine, GLEPP1, human-specific beta 2 microglobin (MG), and rat GAPDH. Lane 1 is the control rat metanephros, lane 2 is hMSCs, and lanes 3-5 are the kidneys formed from three different experiments. It was shown that donor-derived LacZ-positive cells expressed glomerular epithelial cell-specific genes (nephrin, podocine, and GLEPP-1) and renal tubular epithelial cell-specific genes (AQP-1, 1 alpha hydroxylase, PTH receptor 1, and NBC-1) (Fig. 3-3). In contrast to endogenous renal cells, ATP-sensitive K+ channel subunit, Kir6.1/SUR2 (non-patent document 21) expressed in hMSCs was still expressed after relay culture.

### D. Injection and culture of hMSCs in isolated metanephros

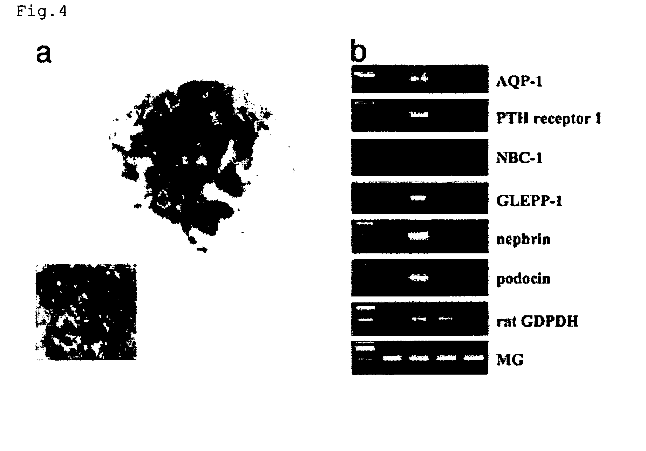

hMSCs which express the LacZ gene through the use of retrovirus were further transduced with GDNF by adenovirus and injected into the cultured metanephros (E13). After 6 days of organ culture, the resulting metanephros were subjected to an X-gal assay (Fig. 4(a)). The inset shows LacZ-positive cells at high magnification. The injected hMSCs-derived cells remain aggregated and do not form high-dimensional kidney structures. After sorting the LacZ-positive cells, RNAs were extracted and were subjected to RT-PCR. Neogenerated kidney primordia before (lane 2) and after (lane 3) organ culturing is shown. A mixture of metanephros and hMSCs before (lane 4) and after (lane 5) organ culture is shown. Lane 1 is a marker (ϕX174/HaeIII). As shown in the figure, even when hMSCs is injected into culture tissue which has already differentiated into metanephros, kidney-specific genes are not expressed (Fig. 4(b)). From the above items, only hMSCs which were injected before the sprouting of ureteric buds could be integrated into the kidney primordias in the organ culture and be transformed to express kidney-specific genes. The gene expression capability can not be achieved under other conditions. That is to say, the above shows that during whole embryo culture, hMSCs complete an initial step essential for commitment to a renal fate and that during organ culture, they further undergo a mesenchyme-to-epithelium transition or differentiation for the creation of stroma.

### E. Therapeutic kidney regeneration in α-gal A deletion Fabry mice

To examine whether the hMSCs-derived nephron is functional, hMSCs were transplanted into an E9.5 embryo of a knockout mouse which does not express the α-gal A gene (Fabry mouse) and a relay culture was carried out (non-patent document 22). This deletion of α-gal A is known in human as Fabry disease causing mainly an abnormal accumulation of sphingoglycolipid (Gb3) in the glomerular epithelial cells and renal tubular epithelial cells, and kidney disorder after birth.

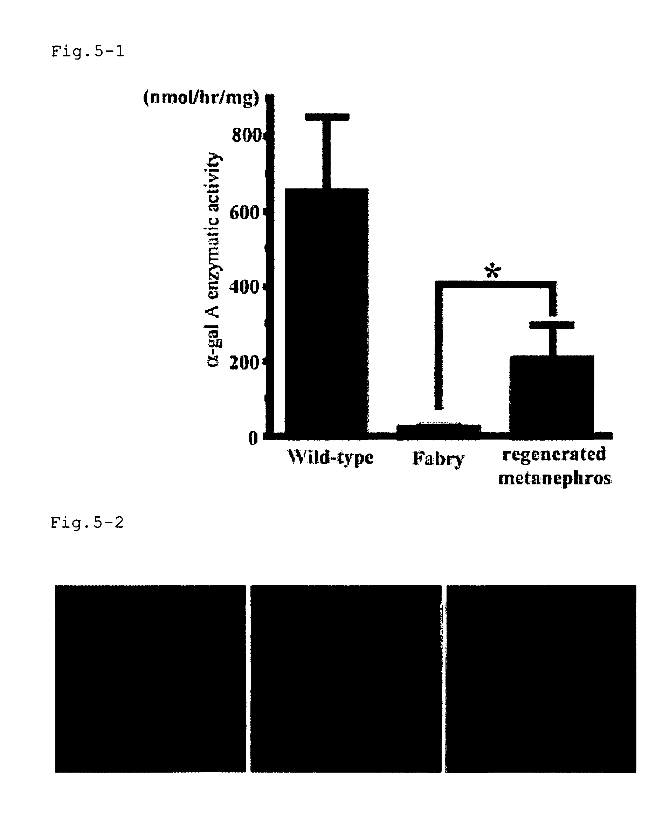

Bioactivity of α-gal A enzyme of the kidney primordial derived from human mesenchymal stem cells, produced by the method described above was evaluated by fluorometry (non-patent document 19). When, as a control, the metanephros of a wild type mouse (left) was compared under the same protocol with that of Fabry mouse (right), the bioactivity of α-gal A in the kidney primordia from the Fabry mouse was extremely low (19.7±5.5 nmol/mg/hour) compared to that from the wild type mouse (655.0±199.6 nmol/mg/hour). However, the kidney primordia having the nephron derived from the injected human mesenchymal stem cells expressed a significantly higher amount of the α-gal A bioactivity (204.2±98.8 nmol/mg/hour, p<0.05, Fig. 5-1) than the wild type mouse.

To confirm the Gb3 clearance capacity of the obtained kidney primordia, an organ culture was carried out in the presence of Gb3, and an analysis was performed by comparing accumulation of Gb3 in the metanephros in the wild type mouse (left) with that in the Fabry mouse (right). It was confirmed that the accumulation of Gb3 in the ureteric bud and S-shaped body (Fig. 5-2 right) in the kidney primordia of the Fabry mouse was markedly cleared by combining with the nephron derived from the human mesenchymal stem cells, formed by the relay culture (Fig. 5-2 center). This result indicates that the newly produced nephron is biologically functioning.

The present invention, described up to this point, revealed that allowing hMSCs to grow in a specific organ region in whole embryo culture can commit hMSCs to the fate of the organ. Injection of GDNF-transduced hMSCs into embryos followed by relay culture makes it possible to create entire nephrons, not just individual kidney structure cells. These hMCS-derived cells are functional as tested by their ability to metabolize Gb3.

hMSCs can be reprogrammed to other fates and organ structures, depending upon the embryonic environment into which they enter. A further advantage of using hMSCs is that although they are of mesodermal origin at the primordial stage, they have the potential to differentiate into cell types that are normally derived from ectoderm or endoderm (non-patent document 23).

The host immune system is not yet fully developed at this stage of the whole embryo culture. Therefore, the host is tolerant to foreign cells. The present invention is to establish a method for generating self-organs from autologous mesenchymal stem cells using the endogenous development system of an immunocompromised foreign host.

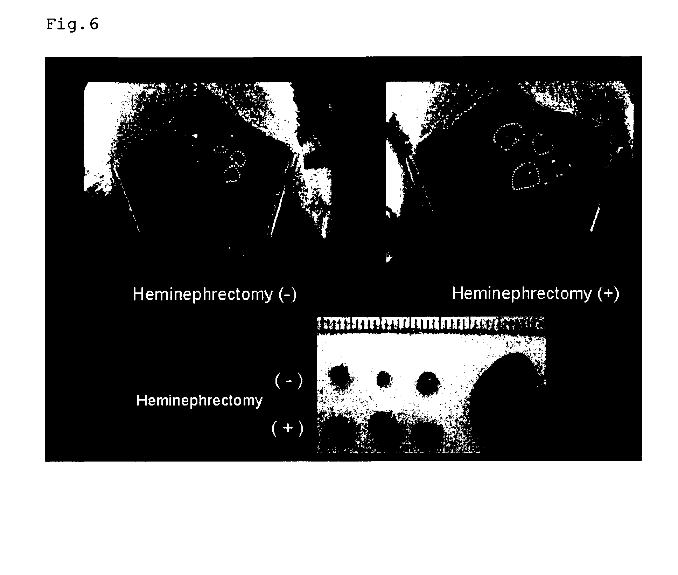

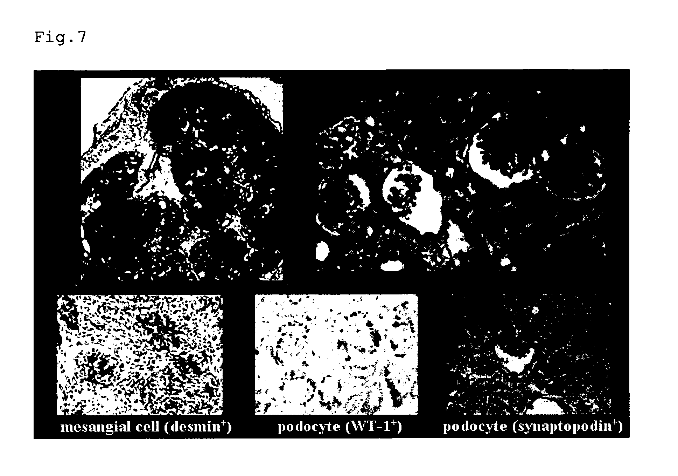

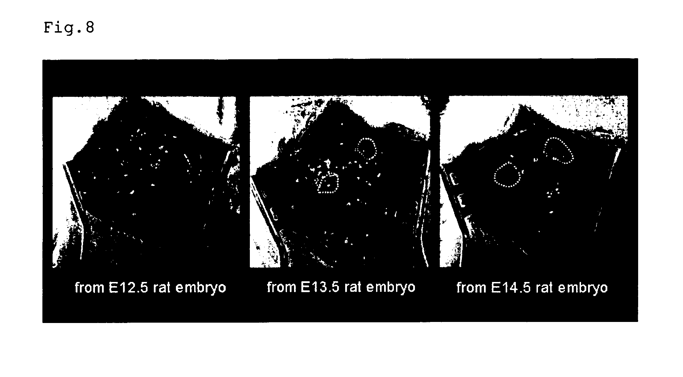

The system described up to this point makes use of the organ culture for the final growth of the kidney primordia, and therefore the kidney formed does not have blood vessels structure. For this reason, the basic function of the kidney, hemofiltration function can not be confirmed, and therefore the system was further improved. It has been reported that the rat metanephros tissue transplanted into the greater omentum continued growing (non-patent document 24). Thus the metanephros tissue was isolated from the E15 embryo, was transplanted into the greater omentum of rat and 2 weeks later laparotomy was performed. It was confirmed that the transplanted metanephros continued growing further in the greater omentum and the blood vessel system from the greater omentum was invading the kidney (Fig. 6). This growth was not decreased even in the state of kidney failure (after resection of one kidney), but on the contrary it was shown to be further accelerated (Fig. 6). A histological analysis of this grown kidney is shown in Fig. 7. Inside of the kidney, blood vessels are filled with erythrocytes that can not be recognized before the transplantation, which show that the blood circulation is histologically opening. In addition, glomerular mesangial cells (desmin positive) and highly differentiated glomerular epithelium cells (WT-1 and synaptopodin positive cells), which could not be confirmed before the transplantation into the greater omentum, were confirmed. Next, to investigate the best timing for the transplantation, the metanephros at various stages was transplanted into the greater omentum (Fig. 8). As in the figure, it was shown that the transplantation of the premature metanephros tissue up to E12.5 did not induce the growth afterwards, but the kidney grew when the metanephros tissue after E13.5 was transplanted.

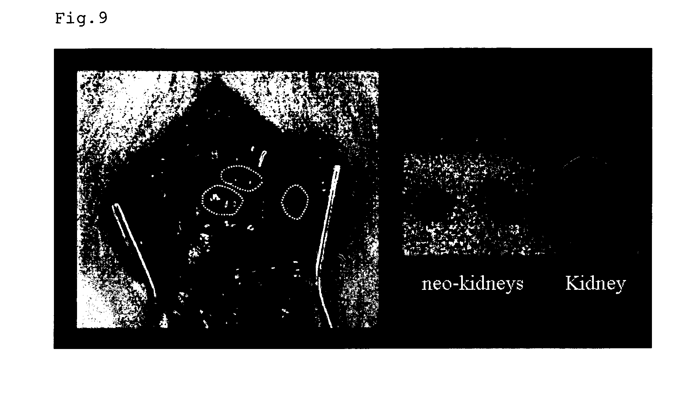

The relay culture was further improved based on above results. That is, a rat embryo (E11.5) was performed through whole embryo culture (48 hours), the organ culture was then performed for 24 hours until reaching the stage where a continuous growth in the greater omentum was possible, and then these were transplanted into the greater omentum (improved relay culture). To further accelerate the growth one kidney was resected. Fig. 9 shows the newly formed kidney which was grown for 2 weeks. It was also confirmed with histological examinations that the blood circulation was opening and the highly differentiated glomerular structure was maintained as mentioned above.

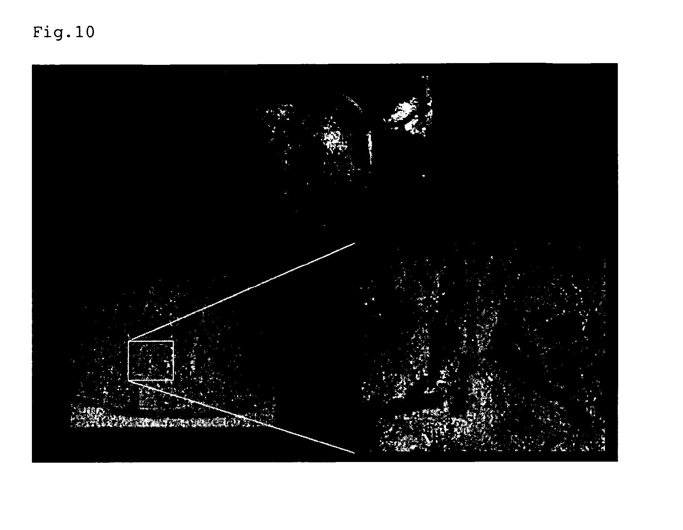

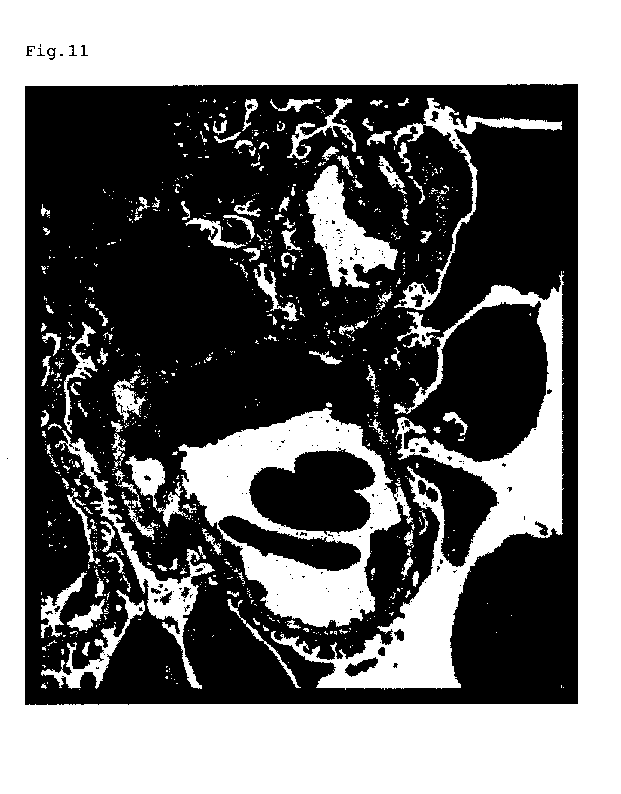

To confirm that this blood was supplied from the blood vessels of the recipient into which transplantation was performed, the kidney primordia was transplanted into the greater omentum of a LacZ rat, the blood vessels of which are stained blue with LacZ. It was shown by macroscopic examination that the blood vessels of the greater omentum were incorporated into the newly formed kidney (Fig. 10, upper), and by tissue staining with LacZ, it was demonstrated that the blood vessels in the kidney were formed by the blue cells derived from the recipient (Fig. 10, lower). The electron microscopy also confirmed the presence of erythrocytes in the glomerular blood vessels, and in addition, the pedicel of highly differentiated glomerular epithelial cells, and the construction of endothelial cells and mesangial cells were confirmed (Fig. 11).

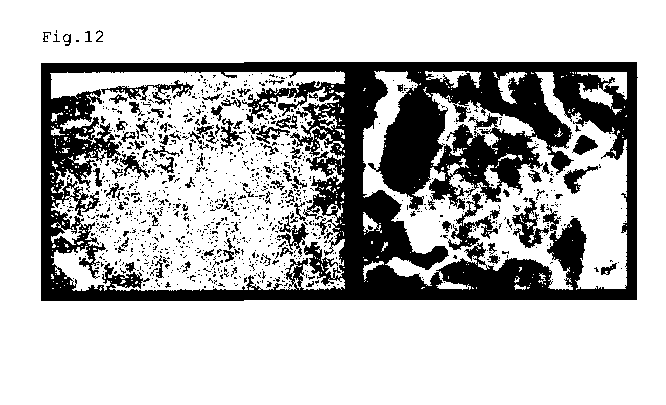

Based on these results, it was examined whether the cloned kidney derived from the human mesenchymal stem cells can produce the recipient's urine by the improved relay culture. The hMSCs, into which the LacZ and GDNF genes introduced using retrovirus and adenovirus, respectively, were injected into the rat embryo (E11.5) at the kidney-forming site. The hMSCs were subjected to whole embryo culture for 24 hours and to organ culture for 24 hours. Fig. 12 is a figure showing the newly formed kidney which was grown in the greater omentum of a LacZ rat using this improved relay culture (2 weeks). It was shown that the renal tubular as well as the blood vascular system inside the kidney were LacZ-positive, and the injected human mesenchymal stem cell-derived nephrons and the recipient-derived blood vessels were integrated.

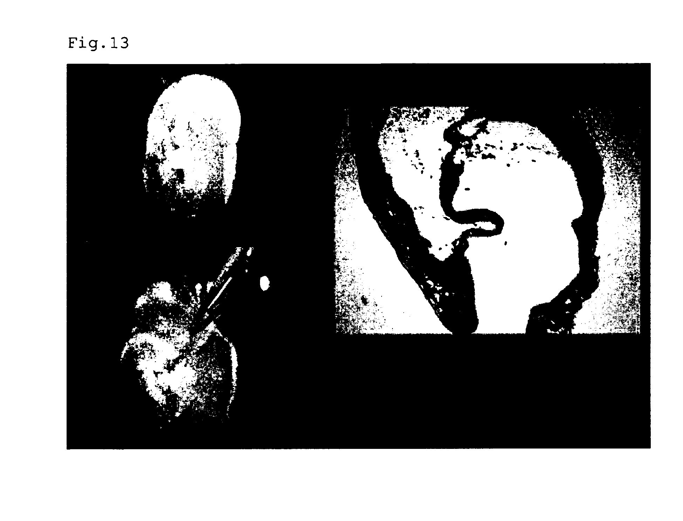

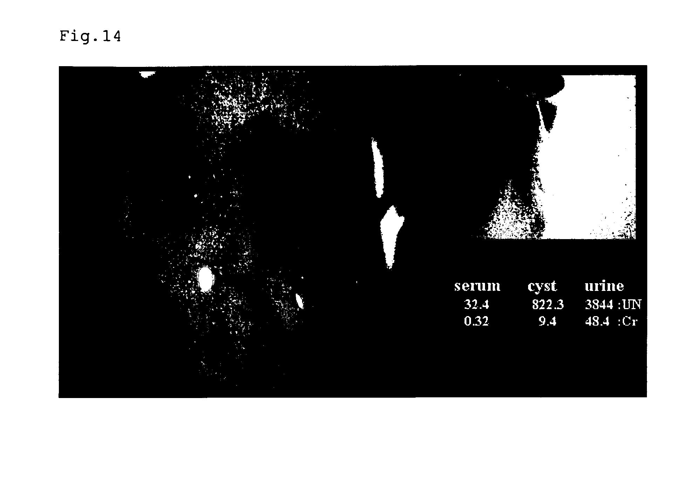

Fig. 13 shows the morphology of the newly formed kidney which was further grown for 4 weeks in the greater omentum. From the image, it was considered that hydronephrosis was caused by urine produced because there was no opening of the ureter in this kidney. Therefore, the liquid retained in the ureter was recovered to test whether this was urine, and it was found that the composition contained significantly higher concentration of urea-nitrogen and creatinine compared with serum, suggesting that it was urine filtered by the glomerulus. Therefore, it is effective to form an outlet for urine by treating the ureter of the cloned kidney to make an opening to the recipient's ureter, bladder, rectum or skin between 2 to 4 weeks when the kidney grows and produces urine. Fig. 14 is a figure showing the urine-like liquid from the new kidney.

### Example 2

Next, in order to differentiate the cells into a ureteric bud-derived collecting tube and ureter, the experiment for locating the transplantation site of hMSCs was performed in the following manner.

### (Materials and methods)

### 1) Chick embryo

The chick sperm eggs (Hyperco) procured from SHIROYAMA POULTRY FARM (Kanagawa Prefecture Tsukui District) were hatched in the incubator, at the temperature of 38°C for 34 to 35.5 hours and the chicks were grown up to a stage of 9 to 11 of Hamburger and Hamilton.

### 2) Determination of transplantation site

Intermediate mesoderm was selected as a transplantation site, the labeled material was introduced into this site, and the movement owing to the development was traced. To determine the transplantation site of the hMSCs, DiI (Molecular Probes) was used as a labeled material. Since this DiI is incorporated into cell membranes of lipophilicity and generates strong fluorescence, it is effective to trace the movement of the labeled cells. The DiI was dissolved with absolute ethanol as it will be about 0.5% (wt/vol), and was further diluted 10 times with 0.3M simple sugar.
Introduction of the labeled material was approached to an embryo by opening a hole to an eggshell, and under a stereoscopic microscope, a small quantity of DiI was then injected into the corresponding site of intermediate mesoderm of a chick embryo in each development stage by using a micropipette without damaging surrounding tissue. Panett-Compton salt solution, which is the normal saline solution used for chick embryo, was properly poured on the embryo to protect its desiccation.
The hole of eggs was closed with Sellotape (registered trademark), and the eggs continued developing inside the incubator kept at 38°C and sufficient humidity environment. Furthermore, the movement of the cells labeled with DiI and the formation process of the ureteric bud were observed with the lapse of time using a fluorescent stereoscopic microscope.

### 3) Results

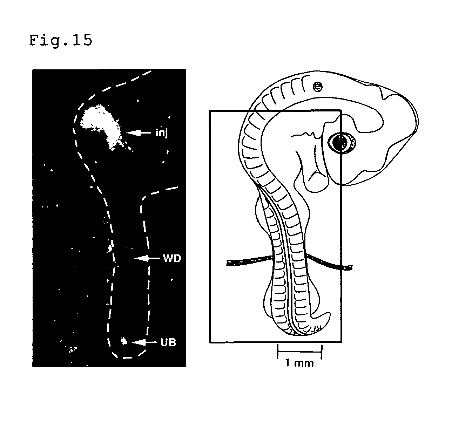

The intermediate mesoderm which was adjacent to the 10th somite at stage 10 (after 35 hours of age), regarded as Wolffian duct primordium, was labeled with DiI, and then the chick embryos continuing development for about 24 hours were immobilized by 4% paraformaldehyde. Furthermore, when the frozen sections produced from them had been observed with a fluorescent microscope, it were confirmed that the DiI was incorporated into the epithelial cells of a Wolffian duct.
Additionally, the present inventors have attempted to alter the stages and sites labeled with DiI in various ways. Thereby, even when the intermediate mesoderm adjacent to the just formed somite from 7 to 12 was labeled at the time of formation of the somite from 7 to 12 (corresponding to approximately stage 9-11), it was confirmed that the DiI was incorporated to a Wolffian duct. It revealed that a Wolffian duct primordium has wide range in terms of time and space than that of the past reports.
When the chick embryos, which had been incorporated the DiI to a Wolffian duct primordium, further continued to be developed for about 24 hours, the incorporation of the DiI into a ureteric bud was observed according to a whole-mount fluorescent stereoscopic microscope image (Fig. 15). It is shown that in this figure, Inj indicates an infusion site of labeled materials, WD indicates a Wolffian duct and UB indicates a ureteric bud.
Based on these results, it is possible to transplant hMSCs into the selected site (the intermediate mesoderm adjacent to the somite 10) at the selected time (stage 10; after about 35 hours of age) in a manner similar as Example 1 and to achieve the differentiation into the target organs.

### Industrial Applicability

The present invention allows a new development in kidney transplantation, for example, allows a patient, such as a dialysis patient with renal disease, to create a freshly generated organ having original functions through transplantation of the isolated autologous mesenchymal stem cells into an embryo inside a pregnant mammalian host or into an embryo dissected from a pregnant mammalian host after growing into a certain level, which is then transplanted into the body of the person himself or herself.

## Claims

1. A method for preparing an organ for transplantation into a mammal by transplanting isolated mammal-derived mesenchymal stem cells into an embryo inside a pregnant mammal host or into an embryo dissected from the pregnant mammal host to induce differentiation of the mesenchymal stem cells, wherein the mesenchymal stem cells are transplanted into intermediate mesoderm of the embryo, at a transplantation time when the host is still at an immunologically tolerant stage.

2. The method according to Claim 1, wherein said organ is a kidney.

3. The method according to Claim 2, wherein said mammal-derived mesenchymal stem cells are differentiated into mesangium cells, tubular epithelial cells, and glomerular epithelial cells by separately transplanting the mesenchymal stem cells into a metanephros-forming mesenchyme.

4. The method according to any one of Claims 1 to 3, wherein said mammal-derived mesenchymal stem cells are differentiatedintothe mammal-derived ureteric bud-derived collecting tube and ureter by transplanting the mesenchymal stem cells into the intermediate mesoderm.

5. The method according to any one of Claims 1 to 4, wherein said host is a mammal having a size of the kidney similar to that of the human kidney.

6. The method according to Claim 5, wherein said host is a pig.

7. The method according to any one of Claims 1 to 6, wherein the mammal-derived mesenchymal stem cells are transplanted into an embryo by transplanting the cells to the host through a transuterine approach.

8. The method according to any one of Claims 1 to 7, wherein the mammal-derived mesenchymal cells are transplanted into an embryo by dissecting the embryo from the uterus and transplanting the cells into the embryo, and then further maturing the embryo in vitro using whole embryo culture.

9. The method according to any one of Claims 1 to 8, wherein the mammal-derived mesenchymal stem cells are human mesenchymal cells.
